# EUROPEAN PATENT APPLICATION

(11) **EP 1 836 995 A1**
(43) Date of publication of application: **26.09.2007**
(21) Application number: 07005951.4
(22) Date of filing: 22.03.2007
(51) Int. Cl.: A61F 2/06, A61F 2/84

(54) **Prothesis with adjustable for side branch access**

(30) Priority: 24.03.2006 US 277427
(71) Applicant: Medtronic Vascular, Inc., Santa Rosa, California 95403 (US)
(72) Inventor: Krivoruchko, Michael, Forestville, CA 95436 (US)
(74) Representative: Zimmermann, Gerd Heinrich

(57) **Abstract**

Tubular prosthesis for deployment in a human body passageway comprises a tubular member adapted for placement in a passageway in a human body. The tubular member comprises a tubular wall having first and second ends and an adjustable side opening in the tubular wall between the first and second ends.

## Description

### FIELD OF THE INVENTION

The invention relates to prosthesis adapted for placement in a passageway in a human body such as an artery and having at least one adjustable opening to provide branch passageway access.

### BACKGROUND OF THE INVENTION

Tubular prostheses such as stents, grafts, and stent-grafts (e.g., stents having an inner and/or outer covering comprising graft material and which may be referred to as covered stents) have been widely used in treating abnormalities in passageways in the human body. In vascular applications, these devices often are used to replace or bypass occluded, diseased or damaged blood vessels such as stenotic or aneurysmal vessels. For example, it is well known to use stent-grafts, which comprise biocompatible graft material (e.g., Dacron ® or expanded, porous polytetrafluoroethylene (ePTFE)) supported by a framework (e.g., one or more stent or stent-like structures), to treat or isolate aneurysms. The framework provides mechanical support and the graft material or liner provides a blood barrier.

Aneurysms generally involve abnormal widening of a duct or canal such as a blood vessel and generally appear in the form of a sac formed by the abnormal dilation of the duct or vessel wall. The abnormally dilated wall typically is weakened and susceptible to rupture. Aneurysms can occur in blood vessels such as in the abdominal aorta where the aneurysm generally extends below the renal arteries distally to or toward the iliac arteries.

In treating an aneurysm with a stent-graft, the stent-graft typically is placed so that one end of the stent-graft is situated proximally or upstream of the diseased portion of the vessel and the other end of the stent-graft is situated distally or downstream of the diseased portion of the vessel. In this manner, the stent-graft extends through the aneurysmal sac and beyond the proximal and distal ends thereof to replace or bypass the dilated wall. The graft material typically forms a blood impervious lumen to facilitate endovascular exclusion of the aneurysm.

Such prostheses can be implanted in an open surgical procedure or with a minimally invasive endovascular approach. Minimally invasive endovascular stent-graft use is preferred by many physicians over traditional open surgery techniques where the diseased vessel is surgically opened, and a graft is sutured into position bypassing the aneurysm. The endovascular approach, which has been used to deliver stents, grafts, and stent grafts, generally involves cutting through the skin to access a lumen of the vasculature. Alternatively, lumenar or vascular access may be achieved percutaneously via successive dilation at a less traumatic entry point. Once access is achieved, the stent-graft can be routed through the vasculature to the target site. For example, a stent-graft delivery catheter loaded with a stent-graft can be percutaneously introduced into the vasculature (e.g., into a femoral artery) and the stent-graft delivered endovascularly across the aneurysm where it is deployed.

When using a balloon expandable stent-graft, balloon catheters generally are used to expand the stent-graft after it is positioned at the target site. When, however, a self-expanding stent-graft is used, the stent-graft generally is radially compressed or folded and placed at the distal end of a sheath or delivery catheter and self expands upon retraction or removal of the sheath at the target site. More specifically, a delivery catheter having coaxial inner and outer tubes arranged for relative axial movement therebetween can be used and loaded with a compressed self-expanding stent-graft. The stent-graft is positioned within the distal end of the outer tube (sheath) and in front of a stop that is fixed to the distal end of the inner tube and has a diameter sized to engage the distal end of the stent-graft as it is being deployed. Once the catheter is positioned for deployment of the stent-graft at the target site, the inner tube is held stationary and the outer tube (sheath) withdrawn so that the stent-graft is gradually exposed and expands. An exemplary stent-graft delivery system is described in U.S. Patent Application Publication No. 2004/0093063, which published on May 13, 2004 to Wright et al. and is entitled Controlled Deployment Delivery System, the disclosure of which is hereby incorporated herein in its entirety by reference.

Although the endovascular approach is much less invasive, and usually requires less recovery time and involves less risk of complication as compared to open surgery, there can be concerns with alignment of non symmetric asymmetric prosthesis features in relatively complex applications such as one involving branch vessels. Branch vessel techniques have involved the delivery of a main device (e.g., a graft or stent-graft) and then a secondary device (e.g., a graft or stent-graft) through a fenestration or side opening in the main device and into a branch vessel.

The procedure becomes more complicated when more than one branch vessel is treated. One example is when an aortic abdominal aneurysm is to be treated and its proximal neck is diseased or damaged to the extent that it cannot support a patent connection with a prosthesis. In this case, grafts or stent-grafts have been provided with fenestrations or openings formed in their side wall below a proximal portion thereof. The fenestrations or openings are to be aligned with the renal arteries and the proximal portion is secured to the aortic wall above the renal arteries.

To ensure alignment of the prostheses fenestrations and branch vessels, current techniques involve placing guidewires through each fenestration and branch vessel (e.g., artery) prior to releasing the main device or prosthesis. This involves manipulation of multiple wires in the aorta at the same time, while the delivery system and stent-graft are still in the aorta. In addition, an angiographic catheter, which may have been used to provide detection of the branch vessels and preliminary prosthesis positioning, may still be in the aorta. Not only is there risk of entanglement of these components, the preformed prosthesis fenestrations may not properly align with the branch vessels due to differences in anatomy from one patient to another. Custom prostheses having preformed fenestrations or openings based on a patient's CAT scans also are not free from risk. A custom design prosthesis is still subject to a surgeon's interpretation of the scan and may not result in the desired anatomical fit. Further, relatively stiff catheters are used to deliver grafts and stent-grafts and these catheters can reshape the vessel (e.g., artery) in which they are introduced. When the vessel is reshaped, even a custom designed prosthesis may not properly align with the branch vessels.

U.S. Patent No. 5,617,878 to Taheri discloses a method comprising interposition of a graft at or around the intersection of major arteries and thereafter, use of intravenous ultrasound or angiogram to visualize and measure the point on the graft where the arterial intersection occurs. A laser or cautery device is then interposed within the graft and used to create an opening in the graft wall at the point of the intersection. A stent is then interposed within the graft and through the created opening of the intersecting artery.

There remains a need to develop and/or improve branch vessel apparatus and methods for endoluminal or endovascular applications.

### SUMMARY OF THE INVENTION

The present invention involves improvements in prosthesis for branch vessel treatment and overcomes disadvantages of prior art. Thus, the above described problems are solved by a tubular prosthesis according to claims 1 and 15. Further aspects, features, details and advantages of the present invention are apparent from the dependent claims, the specification, and the accompanying drawings.

In one embodiment according to the invention, a method of deploying a tubular prosthesis in a passageway in a human body comprises positioning a tubular prosthesis, which has a side wall with an adjustable opening, in a first passageway in a human body with the adjustable opening in the vicinity of a second passageway that branches from the first passageway; and adjusting a dimension of the adjustable opening so that at least a portion of the opening faces the branch passageway and provides fluid flow from the first passageway to the second passageway.

In another embodiment according to the invention, tubular prosthesis for deployment in a human body passageway comprises a tubular member adapted for placement in a passageway in a human body. The tubular member comprises a tubular wall having first and second ends, and an adjustable side opening between the first and second ends.

In another embodiment, tubular prosthesis for deployment in a human body passageway comprises a tubular member adapted for placement in a passageway in a human body. The tubular member comprises a tubular wall, first and second ends, and at least one opening in the tubular wall between the first and second ends. The tubular wall has a member coupled thereto and surrounding at least a portion of the opening. The member, which surrounds at least a portion of the opening, being movable between a closed configuration and a memory set open configuration and tending to move toward the memory set open configuration.

Other features, advantages, and embodiments according to the invention will be apparent to those skilled in the art from the following description and accompanying drawings.

### BRIEF DESCRIPTION OF THE DRAWINGS

FIG. 1 diagrammatically illustrates one embodiment of a tubular prosthesis having an adjustable side opening in accordance with the invention.

FIG. 2 diagrammatically illustrates a bifurcated variation of the prosthesis of FIG. 1 positioned in a passageway in a human body with a pair of adjustable openings in a first closed state.

FIG. 3 diagrammatically illustrates the prosthesis of FIG. 2 with the adjustable openings in an open state.

FIG. 4A illustrates one adjustable opening configuration according to one embodiment of the invention in a closed state.

FIG. 4B illustrates the adjustable opening of FIG. 4A in an open state.

FIG. 5A illustrates the opening mechanism of FIG. 4A biased toward a closed state with a first diameter.

FIG. 5B illustrates the mechanism of FIG. 4A in a free state where it has a second diameter.

FIG. 6 illustrates the adjustable opening configuration of FIG. 4B with a variation on the release mechanism.

FIGS. 7A and 7B illustrate one release mechanism locking device according to one embodiment of the invention.

FIG. 8 diagrammatically illustrates another release mechanism locking device.

FIG. 9 diagrammatically illustrates a further release mechanism locking device.

FIG. 10 illustrates a modular bifurcated stent-graft having adjustable openings in accordance with the invention.

FIG. 10A is a sectional view taken along line 10A-10A in FIG. 10 and illustrates a restraint for a release mechanism.

FIG. 10B illustrates the restraint of FIG. 10A crimped with the release mechanism secured therein.

FIG. 10C is an end view of the ipsilateral leg of FIG. 10.

FIG. 11A illustrates another restraint embodiment in an unlocked state.

FIG. 11B illustrates the embodiment of FIG. 11A in a locked state.

FIG. 11C is a partial sectional view of the locking mechanism depicted in FIG. 11A.

FIG. 11D is a top view taken of the locking mechanism of FIG. 11C.

FIG. 11E is another embodiment of the locking mechanism.

FIG. 12 illustrates a further restraint embodiment in an unlocked state.

### DETAILED DESCRIPTION

The following description will be made with reference to the drawings where when referring to the various figures, it should be understood that like numerals or characters indicate like elements.

The device generally involves a tubular prosthesis (e.g., an arterial graft or stent-graft) having one or more adjustable openings formed in its side wall. The prosthesis, for example, can be positioned in one passageway (e.g., a vessel) in a patient where vessel access to a branch passageway (e.g., a branch vessel) is desired and the adjustable opening enlarged so that the enlarged opening faces and provides fluid flow communication to the branch passageway. When access is desired for a plurality of branch passageways, the prosthesis can include a plurality of such adjustable openings so that each opening can be enlarged to face and provide fluid flow communication with a respective one of the branch passageways. Such access may be required in or around the intersection of a vessel (e.g., the aorta) and other attendant vessels (e.g., major arteries such as the renal, brachiocephalic, subclavian and carotid arteries). ln order to accommodate different combinations or branch vessels, the adjustable openings can be circumferentially spaced from one another and/or spaced from one another in the direction of the longitudinal axis of the prosthesis.

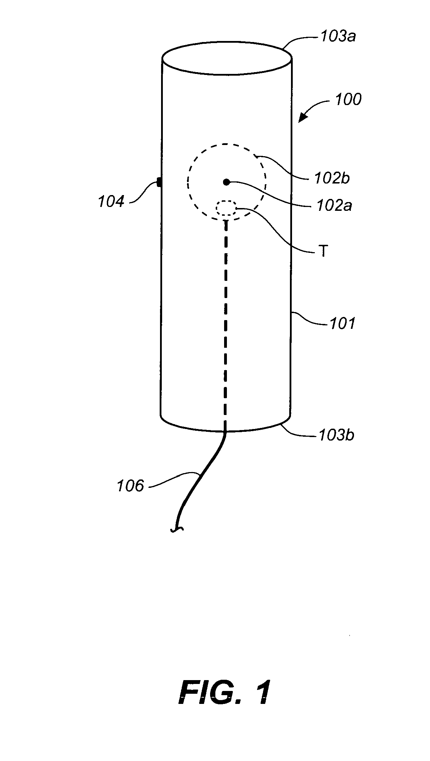

Referring to FIG. 1, a first embodiment of a prosthesis according to the invention is shown and generally designated with reference numeral 100. Prosthesis 100 comprises a tubular member 101 having open ends 103a, b, and an adjustable opening in the side wall thereof which opening has a closed state as depicted with reference number 102a and an open state as depicted in dashed line and indicated with reference numeral 102b. A control member, diagrammatically shown and indicated with reference numeral 106 is coupled to the adjustable opening to control the adjustment or expansion of the adjustable opening so that when the opening is in a closed state such as depicted with numeral 102a and placed in the vicinity of a target branch passageway or vessel "T," which is hypothetically shown in dashed line, it can be expanded to an open state such as depicted with numeral 102b where it encompasses branch passageway "T" and provides fluid flow communication therewith. Alternatively, the expanded opening may simply overlap the passageway if sufficient fluid flow is provided.

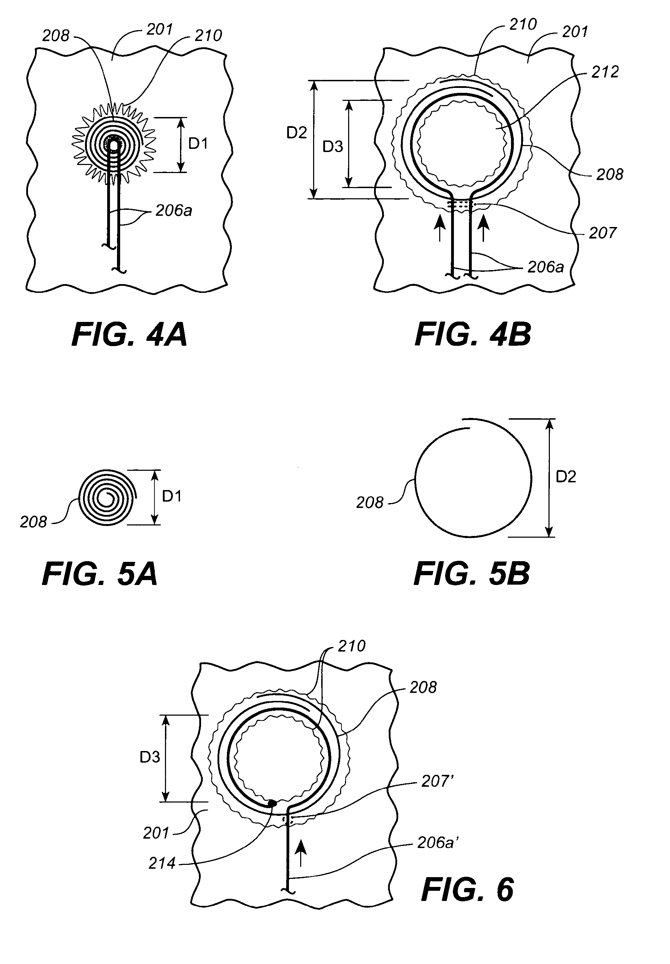

In this example, the control member 106 comprises a flexible member, which can be a string or tether and may be in the form of a suture. Member 106 can be threaded or laced through a channel, which is formed in tubular member 101 and which extends around at least a portion of the adjustable opening. Member 106 can have two end portions or one end portion extending from the channel for manipulation (see e.g., the examples depicted in FIGS. 4A & B and 6). Another member can be placed in or adjacent to the channel to urge the channel and opening toward an open configuration. The end portion or end portions of member 106 then can be drawn or otherwise manipulated to adjust the opening toward closed state 102a or controllably released or otherwise manipulated to allow the opening to move toward open state 102b as will be described in more detail below.

A radiopaque marker 104 can be provided adjacent to the adjustable opening as shown to assist in positioning the adjustable opening in the vicinity of a branch passageway or vessel using fluoroscopic techniques. Marker 104 can be spaced from the adjustable opening center in a circumferential direction and at a predetermined angle so as to provide a direct indication of the opening position. ln the illustrative embodiment, it is spaced about 90 degrees from the opening center. Further, although a single adjustable opening is shown, prosthesis 100 can be provided with a plurality of adjustable openings.

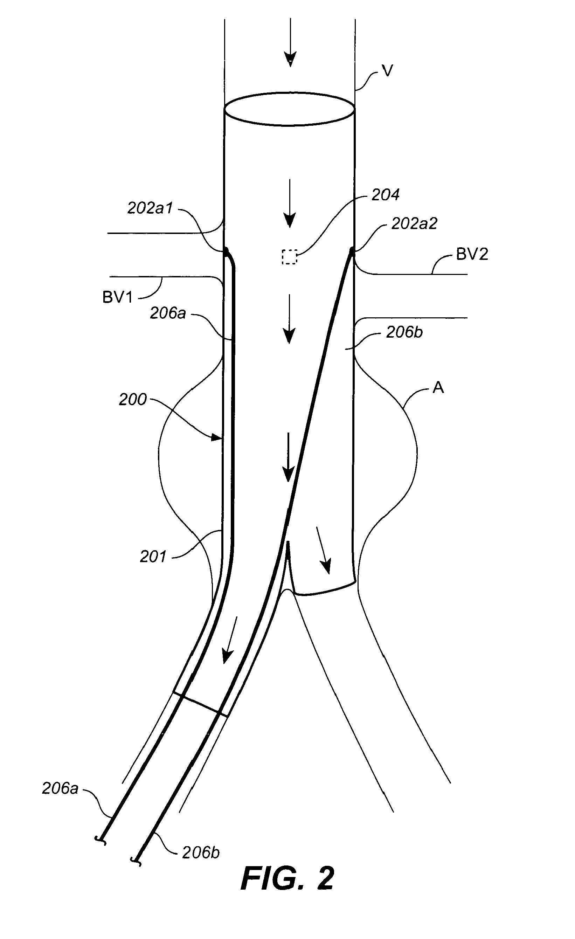

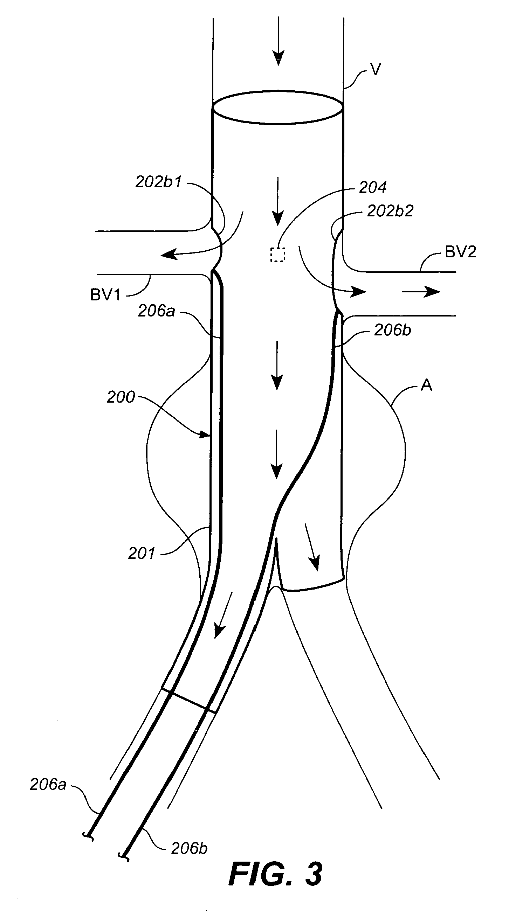

Referring to FIGS. 2 and 3, one example method is shown where a bifurcated prosthesis is deployed in vessel "V," which can be the aorta of a patient, to bypass an aneurysm "A" situated below branch vessels "BV1" and "BV2," which can be renal arteries. It is further noted that in this example, the proximal portion of the prosthesis is secured to the portion of a vessel proximal to the branch vessels due to insufficient proximal neck between the aneurysm and the branch vessels upstream therefrom.

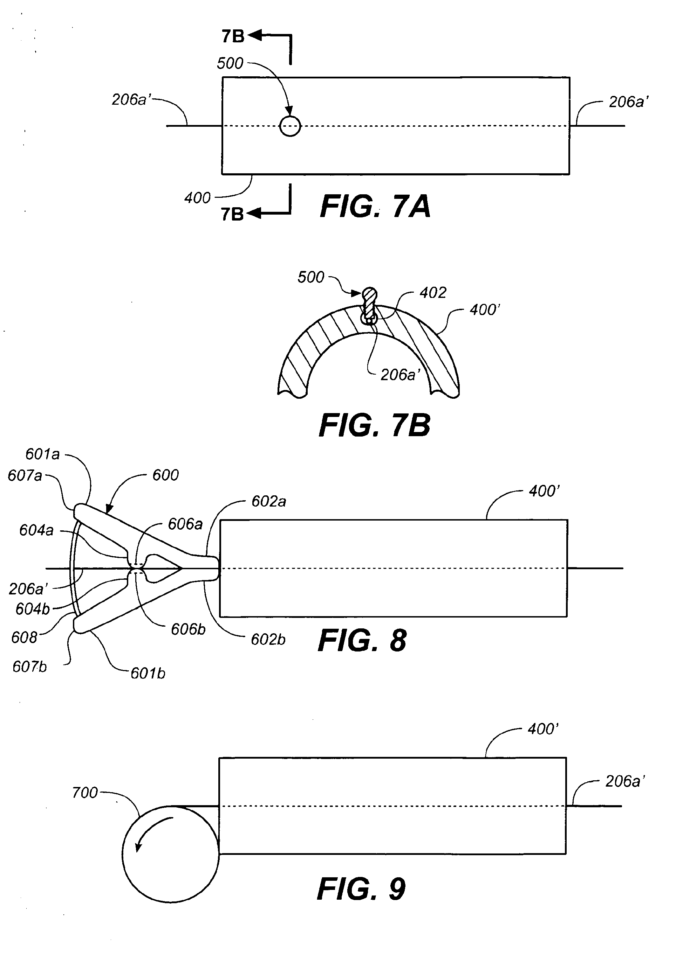

Tubular bifurcated prosthesis 200, which can be an expandable or self-expanding bifurcated graft or stent-graft, is the same as prosthesis 100 with the exception that prosthesis 200 has a bifurcated construction and is shown with two adjustable openings. Accordingly, prosthesis 200 is a bifurcated tubular member 201 having proximal and distal open ends and corresponding adjustable side openings, which can be the same or different (e.g., they can different in size or shape such as circular and elliptical). Each adjustable opening has a closed state 202a1 and 202a2 (FIG. 2) and an open state 202b1 and 202b2 (FIG. 3). Control member 206a and 206b are coupled to a respective one of the adjustable openings to control the adjustment or expansion of a respective opening as will be discussed in more detail below. As will be appreciated from the following description and accompanying illustrations (FIGS. 4A & B), a return portion of each control member is hidden from view. A radiopaque marker 204, which can be similar to marker 104 (FIG 7), is aligned with the diametrically opposed adjustable openings and shown in dashed line as it is hidden from view.

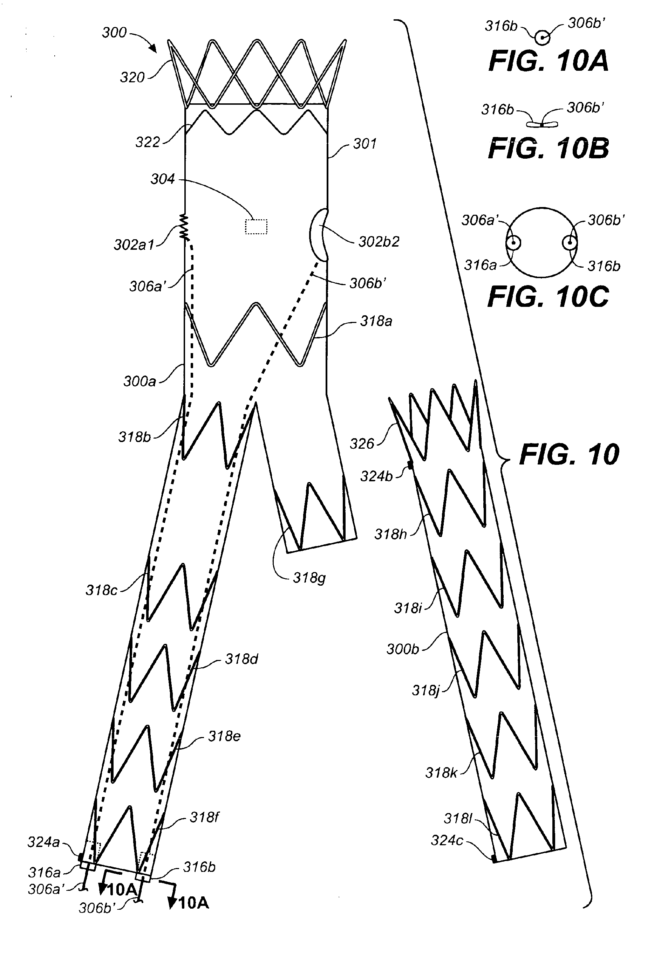

Although not shown, prostheses 100 and 200 can have anchoring mechanisms such as collars having tines extending therefrom or outwardly biased bare springs (see e.g., spring 320 in FIG. 10) at the proximal and/or distal ends thereof to secure the prosthesis to a vessel. Further prostheses 100 and 200 can be in the form of a graft or stent-graft having conventional support wires or stent structure (not shown) positioned so as not to interfere with the adjustable openings.

Prosthesis 200 is delivered endoluminally to a position where it bypasses (or spans) aneurysm "A" in vessel "V" using conventional endovascular graft or stent-graft delivery techniques. Conventional fluoroscopic and intravenous ultrasound device (IVUS) techniques can be used to locate and position the radiopaque marker so that the adjustable openings are positioned in the vicinity of branch vessels "BV1" and "BV2". The proximal portion of the prosthesis is then secured to the portion of vessel "V" proximal to branch vessels "BV1" and "BV2" due to insufficient proximal neck between the aneurysm and the branch vessels upstream therefrom. With the adjustable openings closed, blood flow to the branch vessels is minimized or prevented as depicted with the arrows in FIG. 2.

Referring to FIG. 3, the adjustable opening adjacent to branch vessel "BV1" is allowed to expand so that it faces and provides blood flow to upper branch vessel "BV1" as indicated with the arrow. The prosthesis may have been initially positioned to optimize alignment of the opening and branch vessel. The other adjustable opening is then allowed to expand to where it also faces and provides blood flow to lower branch vessel "BV2." The adjustable opening cooperating with branch vessel "BV2" is allowed to expand further than the other adjustable opening because its center is farther from branch vessel "BV2" as compared to the center of the other opening relative to branch vessel "BV1" (see FIG. 2). In this manner, the prosthesis accommodates differently arranged bifurcations and patients having differing anatomy. If desired, any suitable branch vessel prosthesis (e.g., a stent or stent-graft) can be delivered endoluminally with conventional techniques through the adjustable openings.

Referring to FIGS. 4A and 4B, one embodiment of an adjustable opening will be described in detail. To form a respective adjustable opening, an aperture is formed through a side wall of tubular member 201 and annular channel 212 formed to surround all of or at least a portion of the aperture. Channel 212 can be formed in the tubular member or secured thereto such that it surrounds all or at least a portion of the adjustable opening and defines the adjustable opening. The channel includes channel wall 210, which can be formed from any suitable material such as graft material, which is secured to tubular member 201 with any suitable means (e.g., sewing or suturing). ln the illustrative embodiment, channel wall 210 is sufficiently translucent so as to enable one to view flexible member 206 and split ring 208. Self-expanding split ring 208, which can be a split hoop ring, also is placed in channel 212. Split ring 208 has a radially compressed or closed state with an outer diameter D1 as shown in FIG. 5A (where it is coiled into a plurality of turns and can be referred to as having a spiral configuration) and a free state with a relatively larger diameter D2 as shown in FIG. 5B. Split ring 208 tends to expand the channel diameter and urge the opening toward its open state.

Split ring 208 can be made from any suitable material and can be made from shape memory material and provided with such a preshaped memory set configuration as is known in the art. For example, split ring 208 can be nitinol wire and can be placed in the desired shape (e.g., that shown in FIG. 5B) and heated for about 5-15 minutes in a hot salt bath or sand having a temperature of about 480-515°C. It can then be air cooled or placed in an oil bath or water quenched depending on the desired properties. In one alternative, split ring 208 can be spring steel and preshaped with known techniques to assume the configuration shown in FIG. 5B.

One end portion of flexible member or draw string 206 is laced through channel 212 and positioned adjacent to the inner circumference of channel wall 210 with both ends exiting an opening in the channel wall. The opening can have either of the configurations shown in dashed line in FIGS. 4B and FIG. 6 and indicated with reference numeral 207 and 207', or it can have any other suitable configuration or location. The opening can provide access to the outer surface of tubular member 201 and the draw string extended along the outer surface as shown in FIG. 4A or it can provide access to the inner surface and the draw string extended along the interior of the tubular member as shown in FIG. 10. When the end portions of draw string 206a are drawn, split ring 208 is radially compressed and coils to diameter D1 so that the opening is in a closed state. The channel wall 210 bunches as the string is drawn and the opening closes in a manner similar to closing the opening of a conventional duffle bag with a draw string. When the draw string end portions are manipulated to give the string slack so that the string moves in the direction of the arrows shown in FIG. 4B, split ring 208 is allowed to expand to an intermediate diameter D3 where the adjustable opening is in an open state.

Referring to FIG. 6, a variation is shown where one end of the draw string, indicated here with reference character 206a', is secured to the channel wall or tubular member 201 with the remainder of the string extending through the channel and out from the channel wall opening 207'. In this arrangement, only one string needs to be drawn or released to control the adjustment of the opening.

Any suitable delivery catheter can be used to deliver the prosthesis to the desired site. When the prosthesis is a self-expanding graft or stent-graft, it generally is radially compressed or folded and placed at the distal end of a sheath or delivery catheter and allowed to expand upon deployment from the sheath or catheter at the target site. More specifically, a delivery catheter having coaxial inner and outer tubes arranged for relative axial movement therebetween can be used and loaded with a radially compressed self-expanding prosthesis as described herein. The prosthesis is positioned within the distal end of the outer tube (sheath) and in front of the inner tube. The catheter is routed through the vasculature to the desired site. Once the catheter is positioned for deployment of the prosthesis at the desired site, the inner member, which has a stop connected to a distal end thereof, is held stationary and the outer tube or sheath withdrawn so that the stent-graft is gradually exposed and allowed to expand. The distal stop, which can be in the form of a disk or cylinder, is positioned between the prosthesis and the distal end of the inner member to prevent the stent-graft from moving back as the sheath is withdrawn. The stop therefore is sized to engage the distal end of the stent-graft as the stent-graft is deployed. The proximal ends of the outer tube (sheath) and inner tube are coupled to and manipulated by a handle. The free ends of the draw string 206a (FIG. 4B) or the free end of draw string 206a' (FIG. 6) extend from the prosthesis and pass through the inner tube after which they extend into or through the handle. Any of the stent-graft delivery systems described in U.S. Patent Application Publication No. 2004/0093063, which published on May 13, 2004 to Wright et al. and is entitled Controlled Deployment Delivery System, the disclosure of which is hereby incorporated herein in its entirety by reference, can be used as well.

The stent-graft delivery system handle can include a locking mechanism that releasably locks the draw string(s) and provides controlled release and expansion of the adjustable opening(s). The mechanism also can maintain the adjustable opening in a closed state during delivery.

Referring to FIGS. 7Aand 7B, one locking mechanism or device is shown. In this embodiment, tubular handle 400, which is diagrammatically shown and adapted to be coupled to a catheter delivery system (e.g., one of the delivery systems described above), is provided with a lumen 402 that is formed in its side wall and extends the length thereof and a threaded side bore 404 that therein and extends into lumen 402. Thumb screw 500 is screwed into bore 402 to lock a respective draw string (e.g., 206a') in position. When the thumb screw is rotated the other way, it releases the draw string allows it to move in the direction of the arrows shown in FIGS. 4B and 6 so that the opening can expand. Although a single thumb screw is shown, additional thumb screw, lumen and bore combinations can be provided as desired. For example, when the draw string has two free end portions such as in the example of draw string 206a, a thumb screw, lumen and bore combination can be provided for each free end portion. Further, when more than one adjustable opening is provided and each with a draw string 206a', a thumb screw, lumen and bore combination can be provided for each draw string.

Referring to FIG. 7B, another locking mechanism or locking device is shown. In this embodiment, a clamp 600 in the form of a clothes pin releasably locks a respective draw string (e.g., draw string 206a') in position. Clip 600 comprises two arms 601 a and 601 b and clamping portions 602a and 602b, which are biased toward one another to clamp and lock the draw string in position. Arms 601 a and 601 b include projections 604a and 604b upon which the clip arms pivot when the ends 607a and 607b of the arms opposite the clamping portions are squeezed. A biasing spring 608, which can be in the form of a preshaped metal ribbon or thin, flexible and resilient strip, is coupled to ends 607a and 607b as shown to bias the clip toward a closed position. Clamp projections 604a and 604b have opposed U-shaped open channels formed therein to slidably receive the draw string so that when ends 607a and 607b are squeezed, the clamping portions are urged away from one another and the draw string released. Clamp 600 can be positioned at the proximal end of handle 400' to restrain it from moving in a distal direction. Other clamp configurations can be used as well.

Referring to FIG. 9, a further locking mechanism or device is shown. In this embodiment, draw string is 206a' extends through a central opening in handle 400' and is spooled around reel 700. Reel 700 can have a conventional mechanism (not shown) that to wrap the draw string in the direction of the arrow shown in FIG. 9 and/or provide resistance to the drawing string moving in a distal direction. Reel 700 also can include a conventional release mechanism to allow the draw string to freely move in the distal direction.

Other locking mechanisms also can be used. For example, lever arms can be pivotally mounted to the housing. Each arm can have a projection that extends into a lumen such as lumen 402 to frictionally lock the draw string in position when the arm is pivoted toward the housing.

Referring to FIG. 10, another prosthesis in accordance with the invention and generally designated with reference numeral 300 is shown. Prosthesis 300 is a bifurcated stent-graft having a modular construction, which includes portions 300a (having and ipsilateral leg portion) and 300b (the contralateral leg portion) to facilitate delivery and deployment at a bifurcated passageway such as where the aorta branches to the iliac arteries. The contralateral leg portion 300b can be coupled to main portion 300a in situ as is as is known in the art.

Stent-graft portion 300a comprises a bifurcated tubular member 301 comprising any suitable graft material and annular undulating wire spring elements or stents 318a-g, which structurally support bifurcated tubular graft 301 as is conventional in the art. Tubular graft portion 301 can be positioned on the interior and/or exterior of the wire spring elements 318a-g, which can be secured thereto with any suitable means such as sutures. The prosthesis also includes undulating wire support spring 322 at the proximal end thereof to provide radial strength. Spring 322 also can be positioned on the interior and/or exterior of tubular graft 301. Another such wire support spring (not shown) can be provided along the distal end portion of the ipsilateral leg portion of tubular graft 301. Bare spring 320 is secured to the proximal end of tubular graft portion 301. Bare spring 320 has an undulating configuration and a radially outward bias when in a free state (e.g., a relaxed state). In this manner, spring 320 serves to help secure the proximal end of the stent graft against the wall of the lumen in which the prosthesis is to be placed. It should be understood, however, that other anchoring means can be used in lieu of spring 320 or in combination with one or more springs 320. The spring elements, support springs and bare springs, which may in any combination be incorporated into any of the embodiments described herein, can be of any suitable material as would be apparent to one of ordinary skill in the art. One suitable material is nitinol. The graft material for any of the prostheses described herein also can be any suitable material such as Dacron ® or expanded polytetrafluoroethylene (ePTFE).

Tubular member 301 includes first and second adjustable openings, one of which is shown in a closed state (adjustable opening 302a1) and one which is shown in an open state (adjustable opening 302b2). The openings are constructed in the same manner as the adjustable opening shown and described in connection with FIG. 6. A radiopaque marker 304, which can have the shape shown or any other suitable shape, can be positioned between the adjustable openings to assist in fluoroscopic positioning of the adjustable openings during prosthesis delivery. The marker can be secured to the prosthesis with sutures or any suitable biocompatible adhesive. Draw strings or tethers 306a' and 306b' provide controlled expansion of the openings in the same manner as tether or draw string 206a' provides such control in the embodiment shown in FIG. 6. The distal end of the ipsilateral leg portion further includes two deformable tubes 316a,b, which can be diametrically opposed within tubular graft portion 301 (see e.g., FIG. 10C) and secured thereto with any suitable means such as biomedical adhesive. Draw strings 306a' and 306b', which can structurally correspond to drawing sting 206a' and can be coupled to the graft in the same manner, pass through tubes 316a,b and are slidably disposed therein when the tubes are open as shown in FIG. 10A. In this manner, each string can be controllable released until the desired opening expansion is achieved. Then the tubes can be crimped as shown in FIG. 10B using conventional endoscopic instruments to prevent further expansion of the opening. The portions of the draw strings downstream from tubes 316a,b can then be cut and removed with conventional endoscopic instruments as well.

Contralateral leg portion 300b comprises a tubular graft member and annular wire springs or stents 318h-l, which can be secured to the graft member in the same manner that springs 318a-g are coupled to bifurcated tubular graft member 301. Further, graft material can extend into the apices of the proximal spring 326 of the contralateral leg as shown in FIG. 10. Proximal spring 326 can be biased radially outward to enhance the connection to the contralateral portion of bifurcated tubular member 301 when inserted therein.

Additional radiopaque markers 324a,b,c also can be provided to facilitate positioning the stent-graft portions at the desired location. Such markers also can be secured to the prostheses with any suitable means. Further, the proximal end of the prosthesis also can be scalloped (like petals) or provided with a cutout as can any of the prosthesis described herein when suitable for the intended application. For example, when the prosthesis is used to bypass an abdominal aortic aneurysm and its proximal portion placed above the renal arteries, a cutout can be provided to allow blood flow to the superior mesentery artery.

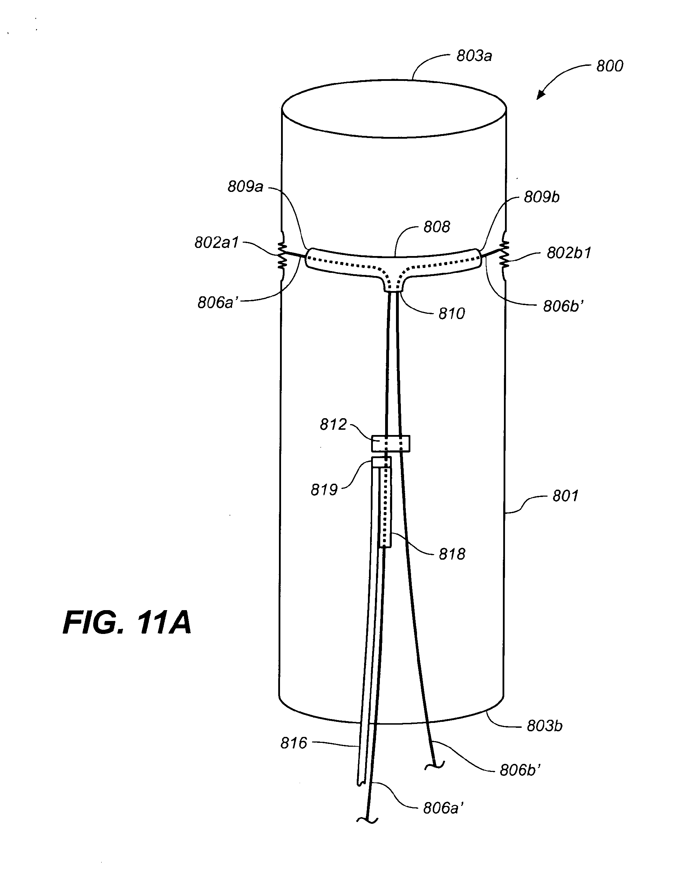

Referring to FIGS. 11A-D, another prosthesis embodiment designated with reference numeral 800 is shown illustrating a further draw string locking system or restraint that can serve as an alternative arrangement to tubes 316a, b. Prosthesis 800 comprises tubular member 801 having open ends 803a,b and adjustable openings formed in the side wall thereof. The adjustable openings are depicted in a closed state 802a1 and 802b1 in FIG. 11A and in an open state 802b1 and 802b2 in FIG. 112B.

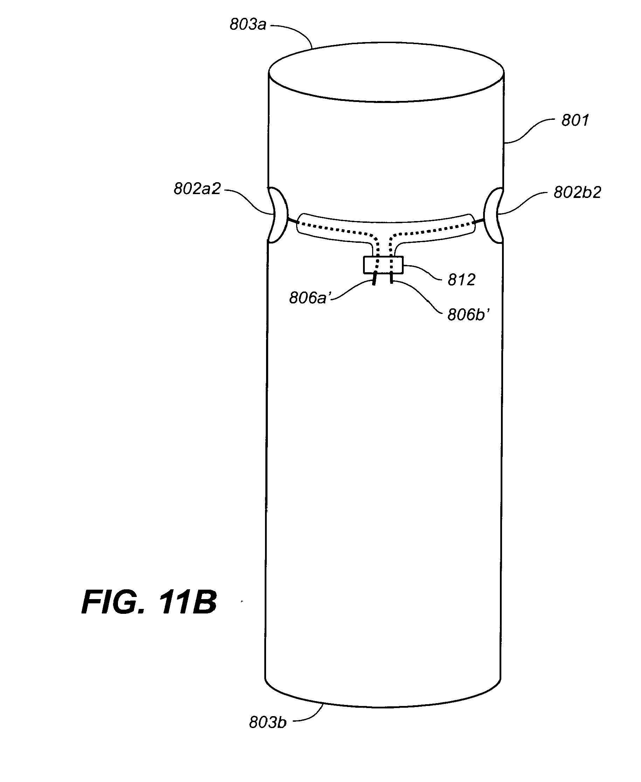

Referring to FIG. 11A, circumferentially extending tube 808, which has end openings 809a and 809b and a central portion opening 810, is secured to tubular member 801 between the adjustable openings with conventional means such as adhesive or sutures. Draw strings 806a' and 806b', which can structurally correspond to drawing string 206a' and can be coupled to the graft in the same manner, extend from adjustable openings 802a1 and 802a2, which can structurally correspond to any of the adjustable openings described above and are shown in a closed state, into inlets 809a and 809b of circumferentially extending tubular member 808, and out from outlet 810 of tubular member 801. The draw strings are slidably disposed in tube 808 so that the adjustable openings can be controllably expanded or otherwise adjusted. For example, each string can be controllable released until the desired opening expansion is achieved and then locked in position. In the illustrative embodiment, draw strings 806a' and 806b' are disposed in sliding locking device 812. Pusher tube 818, which is secured to catheter 816, is coupled to one of the draw strings (e.g., draw string 806a') so that it can be advanced therealong until it engages tube 808 where it prevents further release of the draw strings and expansion of the adjustable opening (FIG. 11 B). The catheter and pusher are withdrawn and portions of the draw strings downstream from assembly 812 cut and removed with conventional endoscopic instruments as shown in FIG. 11 B.

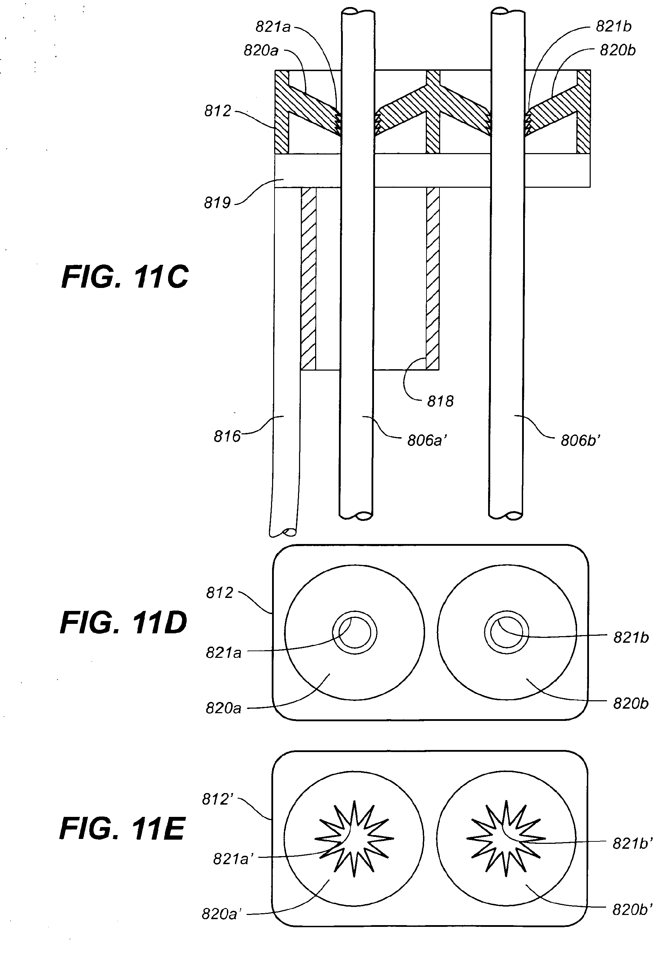

Referring to FIGS. 11C and D, the locking assembly will be described in more detail. The locking assembly includes locking device 812, which comprises a housing that supports or in which are formed funnel shaped portions or members 820a,b. Each member 820a,b has a center opening into which a respective draw string 806a' or 806b' snugly fits. The housing, which is shown in FIG. 11 D with a rectangular configuration, is sized with an engagement surface that is larger that opening 810 so that tube 808 forms a stop for the housing. Each member 820a,b has teeth-like annular ribs or ridges 821 a and 821 b, respectively, along its inner circumference. The rib apices or edges are oriented to limit the draw strings to moving therethrouugh only in one direction and in the illustrative embodiment point proximally at an acute angle relative to the center axis of a center opening in a respective member 820a,b. In other words, the ribs allow the locking device to move distally along the draw strings (i.e., toward tubular member 808), but prevent the draw strings from moving distally (i.e., toward tubular member 808) once locking device 812 is in abutment with tube 808. In this manner, the locking device prevents the draw strings from being further released after the device 812 is positioned against tubular member 808. Pusher member 818, which is tubular in the illustrative embodiment and surrounds one of the draw strings for guidance therealong, can be provided with an optional element or block 819 that is configured to extend over the lower surface of locking device 812 and has a pair of bores or openings (not shown) for slidably receiving the draw strings.

Referring to FIG. 11 E, another locking device is shown and indicated with reference numeral 812', which comprises a pair of funnel shaped members 820a' and 820b' supported or formed therein. Each member 820a' and 820b' has a center opening for receiving a respective draw string a plurality of radially extending teeth 821 a' and 821 b', which extend proximally and toward the center axis of their respective members 820a' and 820b' at an acute angle in a similar manner as ridges 820a and 820b.

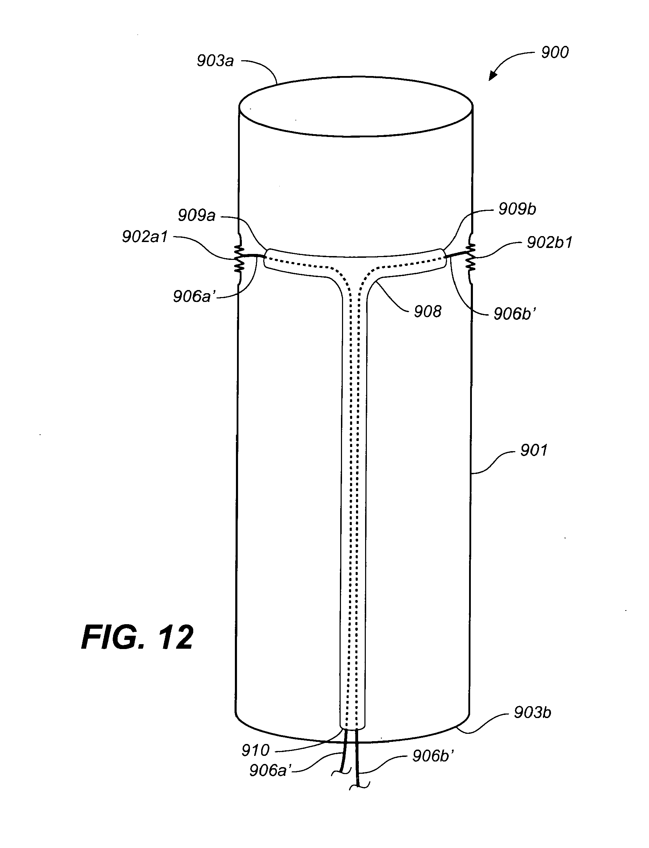

Referring to FIG. 12, a further embodiment is shown. In this embodiment, prosthesis 900, which includes a tubular graft 901 having first and second open ends 903a,b, and adjustable openings 902a1 and 902b1, which are shown in a closed state and which can be structurally the same as adjustable openings 802a1 and 802a2. Draw string tube 908 has a T-shaped configuration with inlets 909a,b and outlet 910. Draw strings 906a' and 906b, which can structurally correspond to draw strings 806a' and 806b', extend from respective adjustable openings and into respective inlets 909a,b of the circumferentially extending portion of tubular member 908. Tubular member 908 can be secured to tubular member 901 with any suitable means such as adhesive or sutures. The draw strings extend from that portion proximally and exit outlet opening 910 where a locking assembly including a locking device 812, catheter 816 and a pusher member 818 as described above can be used to lock the adjustable openings at the desired size. The portions of the drawstrings proximal to the locking device can be cut and removed with endovascular instruments as described above. Member 819 also can be used as described in connection with prosthesis 800.

Although a locking device having a pair of locking elements has been described, the locking elements can be separate and a pusher tube or member provided for each locking element.

Tubular members 801 and 901 can comprise any suitable graft material such as Dacron ® or expanded polytetrafluoroethylene (ePTFE). Any suitable material (e.g., one that can provide a low profile) can be used for tubes 808 and 908 such as the same graft material used to form tubes 801 and 901. Further, any desired stent configuration can be used in either prosthesis 800 or 900 such as the undulating configuration shown in FIG 10 to provide further support for tubes 801 and/or 809. One or more bare crown springs similar to spring 320 and/or one or more support springs similar to spring 322 also can be used. The prostheses also can have a modular bifurcated construction as well.

Any feature described in any one embodiment described herein can be combined with any other feature of any of the other embodiments whether preferred or not.

Variations and modifications of the devices and methods disclosed herein will be readily apparent to persons skilled in the art.

## Claims

1. Tubular prosthesis (100, 200) for deployment in a human body passageway comprising a tubular member (101, 201) adapted for placement in a passageway in a human body, said tubular member (101, 201) comprising a tubular wall having first and second ends (103a, 103b) and an adjustable side opening (102a, 102b, 202a, 202b) in said tubular wall between said first and second ends.

2. The prosthesis of claim 1 wherein said tubular member has a channel (212) surrounding at least a portion of said opening.

3. The prosthesis of claim 2 further including a self-expanding member (208) disposed in said channel (212).

4. The prosthesis of claim 3 wherein said self-expanding member (208) has a relaxed open configuration and a closed configuration and tends to move toward said open configuration, said self-expanding member (208) having a diameter (D1) when in said closed configuration that is less than the diameter (D2) of said self-expanding member (208) when in said open configuration.

5. The prosthesis of claim 4 further including a flexible member (206), said flexible member (206) extending through said channel (212) and having two end portions (206a) extending out from said channel (212), whereby said flexible member (206) can be provided slack when said self-expanding member is in said closed position to allow said self-expanding member to expand toward said open configuration.

6. The prosthesis of claim 4 further including a flexible member (206'), said flexible member (206') extending through said channel (212) and having one end portion (206a') extending out from said channel and another end portion (214) secured to said tubular member, whereby said flexible member (206') can be provided slack when said self-expanding member (208) is in said closed position to allow said self-expanding member (208) to expand toward said open configuration.

7. The prosthesis of any of claims 4 to 6 wherein said prosthesis has a second adjustable opening in said tubular wall between said first and second ends.

8. The prosthesis of claim 7 wherein said tubular member has a second channel surrounding at least a portion of said second opening.

9. The prosthesis of claim 8 further including a second self-expanding member disposed in said second channel.

10. The prosthesis of claim 9 wherein said second self-expanding member has a relaxed open configuration and a closed configuration, the diameter of said second self-expanding member in said closed configuration being less its diameter when in said open configuration.

11. The prosthesis of claim 10 further including a second flexible member, said second flexible member extending through said second channel and having two end portions extending out from said second channel, whereby said second flexible member can be provided slack when said second self-expanding member is in said closed position to allow second self-expanding member to expand toward said open configuration.

12. The prosthesis of claim 10 further including a second flexible member, said second flexible member extending through said second channel and having one end portion extending out from said second channel and another end portion secured to said tubular member, whereby said second flexible member can be provided slack when second self-expanding member is in said closed position to allow second self-expanding member to expand toward said open configuration.

13. The prosthesis of any of the preceding claims wherein said prosthesis is a tubular graft.

14. The prosthesis of any of the preceding claims wherein said prosthesis is a stent-graft.

15. Tubular prosthesis for deployment in a human body passageway comprising a tubular member adapted for placement in a passageway in a human body, said tubular member comprising a tubular wall, first and second ends, and at least one opening in said tubular wall between said first and second ends, said tubular wall having a member coupled thereto and surrounding at least a portion of said opening, said member, which surrounds at least a portion of said opening, being movable between a closed configuration and a memory set open configuration and tending to move toward said memory set open configuration.

16. The prosthesis of claim 15 wherein said tubular wall includes a second opening in said tubular wall between said first and second ends, said tubular wall having another member coupled thereto and surrounding at least a portion of said second opening, said another member, which surrounds at least a portion of said second opening, being movable between a closed configuration and a memory set open configuration and tending to move toward said memory set open configuration.

17. The prosthesis of claim 16 wherein said openings each have an open and closed configuration, and further including a plurality of flexible members coupled to said tubular wall, one flexible member surrounding at least a portion of one of said openings and the other flexible member surrounding at least a portion of the other of said openings so that each opening can be drawn toward said closed configurations.

18. The prosthesis of claim 16 wherein said opening has an open configuration and a closed configuration, and further including a flexible member coupled to said tubular wall and surrounding at least a portion of said opening so that the opening can be drawn toward said closed configuration.

19. The prosthesis of any of claims 15 to 18 wherein said prosthesis is a tubular graft.

20. The prosthesis of any of claims 15 to 19 wherein said prosthesis is a stent graft.
